# EUROPÄISCHE PATENTSCHRIFT

(11) **EP 0 664 830 B1**
(45) Veröffentlichungstag und Bekanntmachung des Hinweises auf die Patenterteilung: **29.01.1997**
(21) Anmeldenummer: 93922519.9
(22) Anmeldetag: 06.10.1993
(51) Int. Cl.: C11D 1/94

(54) **WÄSSRIGE DETERGENSGEMISCHE**
AQUEOUS DETERGENT MIXTURES
MELANGES AQUEUX DE DETERGENTS

(30) Priorität: 14.10.1992 DE 4234487
(43) Veröffentlichungstag der Anmeldung: 02.08.1995
(73) Patentinhaber: Henkel Kommanditgesellschaft auf Aktien, 40191 Düsseldorf (DE)
(72) Erfinder: GIESEN, Brigitte, D-40235 Düsseldorf (DE); KREIENFELD, Günter, D-40589 Düsseldorf (DE); SYLDATH, Andreas, D-40589 Düsseldorf (DE); SCHMID, Karl-Heinz, D-40822 Mettmann (DE)
(86) Internationale Anmeldenummer: EP9302727
(87) Internationale Veröffentlichungsnummer: WO9409102

(56) Entgegenhaltungen:
- EP-A- 0 232 153
- EP-A- 0 341 071
- WO-A-91/11506
- US-A- 4 668 422

## Beschreibung

### Gebiet der Erfindung

Gegenstand der Erfindung sind wäßrige Detergensgemische enthaltend Alkyl- und/oder Alkenyloligoglykoside, Alkylsulfate, Alkylethersulfate und amphotere bzw. zwitterionische Tenside, Handgeschirrspülmittel, die diese Gemische enthalten sowie die Verwendung der Gemische zur Herstellung von oberflächenaktiven Mitteln.

### Stand der Technik

Alkyloligoglykoside und insbesondere Alkyloligoglucoside stellen nichtionische Tenside dar, die wegen ihrer nativen Rohstoffbasis - Fettalkohol und Zucker - zunehmend an Bedeutung gewinnen und beispielsweise in manuellen Spülmitteln oder kosmetischen Produkten eingesetzt werden [vgl. **Tens. Surf.Det. 28, 413 (1991)**]. Trotz guter anwendungstechnischer Ergebnisse besteht dennoch das Bedürfnis nach Detergensmischungen auf Basis von Alkylglucosiden, deren Leistungsvermögen das der Einzelstoffe in synergistischer Weise übersteigt.

In der Vergangenheit hat es nicht an Versuchen gemangelt, Detergensgemische auf Basis von Alkyloligoglucosiden zu entwickeln, die über vorteilhafte Eigenschaften verfügen.

Aus den Patentschriften **EP-B-0 070 074, EP-B-0 070 075, EP-B-070 076** und **EP-B-0 070 077** (Procter & Gamble) sind beispielsweise schaumstarke Kombinationen von Alkyloligoglucosiden mit anionischen Tensiden, wie Seifen, Alkylbenzolsulfonaten, Fettalkoholsulfaten, konventionellen Fettalkoholethersulfaten, alpha-Olefinsulfonaten und Alkansulfonaten sowie gegebenenfalls Betaintensiden bekannt.

In der Deutschen Offenlegungsschrift **DE-A1 35 34 082** werden ferner hautfreundliche Geschirrspülmittel vorgeschlagen, die eine Kombination anionischer Sulfat- bzw. Sulfonattenside, Alkyloligoglucoside und Fettsäurealkanolamide enthalten.

Aus der intemationalen Patentanmeldung **WO 91/11506** sind wäßrige Detergensgemische bekannt, die 10 Gew.-% Alkylglucoside, 30 bis 40 Gew.-% Alkylsulfate, 40 bis 50 Gew.-% Alkylethersulfate und 10 Gew.-% Alkyldimethylacylamidobetaine enthalten. Die Mischungen werden vorzugsweise für die Herstellung von manuellen Geschirrspülmitteln eingesetzt.

Bei Einsatz dieser bekannten Detergensmischungen. in oberflächenaktiven Mitteln müssen jedoch bislang in manchen Fällen Abstriche hinsichtlich des Leistungsvermögens und der ökotoxikologischen Verträglichkeit in Kauf genommen werden.

Die Aufgabe der Erfindung bestand somit darin, Detergensgemische auf der Basis von Alkyl- und/oder Alkenyloligoglykosiden mit weiter verbesserten Eigenschaften zu entwickeln.

### Beschreibung der Erfindung

Gegenstand der Erfindung sind wäßrige Detergensgemische enthaltend - jeweils bezogen auf den Feststoffgehalt -
a) 12 bis 15 Gew.-% Alkyl- und/oder Alkenyloligoglykoside,
b) 20 bis 30 Gew.-% Alkylsulfate,
c) 40 bis 60 Gew.-% Alkylethersulfate und
d) 12 bis 15 Gew.-% amphotere bzw. zwitterionische Tenside.

Überraschenderweise wurde gefunden, daß die erfindungsgemäßen Detergensgemische über ein Wasch-, Spül-, Schaum- und Reinigungsvermögen sowie eine hautkosmetische Verträglichkeit verfügen, die die der Einzelstoffe im Sinne einer synergistischen Verstärkung übertrifft.
a) **Alkyl- und/oder Alkenyloligoglykoside (APG)** stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.
   Die Alkyl- und/oder Alkenyloligoglykoside folgen der Formel (I),

   R¹-O-[G)ₚ (I)

   in der R¹ für einen linearen oder verzweigten Alkyl-oder Alkenylrest mit 4 bis 22 Kohlenstoffatomen, [G] für eine Glykoseeinheit mit 5 oder 6 Kohlenstoffatomen und p für eine Zahl von 1 bis 10 steht.
   Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkylund/oder Alkenyloligogl**ucoside**.
   Die Indexzahl p in der allgemeinen Formel (I) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.
   Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂- Alkohol verunreinigt sein können sowie Alkyloligoglucoside auf Basis technischer C_{9/11}-Oxoalkohole (DP = 1 bis 3).
   Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
b) **Fettalkoholsulfate (FAS)**, die im Sinne der Erfindung Verwendung finden können, folgen der Formel (II),

   R³O-SO₃X (II)

   in der R³ für einen linearen oder verzweigten Alkyloder Alkenylrest mit 6 bis 22 Kohlenstoffatomen und X für ein Alkali- oder Erdalkalimetall steht.
   Auch bei diesen Stoffen handelt es sich um bekannte chemische Verbindungen, die durch Sulfatierung von Fettalkoholen erhalten werden können. Typische Beispiele sind die Sulfate von Capronalkohol, Caprylalkohol, Caprinalkohol, Laurylalkohol, Myristylalkohol, Cetylalkohol, Palmoleylalkohol, Stearylalkohol, Oleylalkohol, Elaidylalkohol, Petroselinylalkohol, Arachylalkohol, Gadoleylalkohol, Behenylalkohol und Erucylalkohol sowie deren technische Gemische. Vorzugsweise werden Sulfate von technischen C_{12/18}-Kokosfettalkohol- bzw. C_{16/18}-Talgfettalkoholschnitten in Form ihrer Natriumsalze eingesetzt.
c) **Fettalkoholetheraulfate (FES)**, die im Sinne der Erfindung Verwendung finden können, folgen der Formel (III),

   R³O-(CH₂CH₂O)ₙ-SO₃X (III)

   in der R³ für einen linearen oder verzweigten Alkyloder Alkenylrest mit 6 bis 22 Kohlenstoffatomen, n für Zahlen von 1 bis 10 und X für ein Alkali- oder Erdalkalimetall steht.
   Auch bei diesen Stoffen handelt es sich um bekannte chemische Verbindungen, die durch Sulfatierung von Fettalkoholpolyglycolethern erhalten werden können. Vorzugsweise werden FES mit eingeengter Homologenverteilung (NRE = narrow range ethoxylates) eingesetzt, wie sie beispielsweise in der Internationalen Patentanmeldung **WO 91/05 764** sowie in der Übersicht von D.L.Smith in **J.Am. Oil.Chem.Soc. 68, 629 (1991)** beschrieben werden.
   Typische Beispiele sind die Sulfatierungsprodukte von Addukten von 1 bis 10 Mol Ethylenoxid (konventionelle oder eingeengte Homologenverteilung) an jeweils 1 Mol Capronalkohol, Caprylalkohol, Caprinalkohol, Laurylalkohol, Myristylalkohol, Cetylalkohol, Palmoleylalkohol, Stearylalkohol, Oleylalkohol, Elaidylalkohol, Petroselinylalkohol, Arachylalkohol, Gadoleylalkohol, Behenylalkohol und Erucylalkohol sowie deren technische Gemische. Bevorzugt sind Sulfate von Addukten von 2 bis 7 Mol Ethylenoxid an gesättigte Kokosfettalkohole mit 12 bis 18 Kohlenstoffatomen in Form ihrer Natrium-, Kaliumund/oder Magnesiumsalze. Vorzugsweise werden Fettalkoholethersulfate eingesetzt, die sich von entsprechenden Fettalkoholpolyglycolethern ableiten, die ihrerseits in Gegenwart von calciniertem oder insbesondere hydrophobiertem Hydrotalcit hergestellt worden sind und daher eine besonders vorteilhaft eingeengte Homologenverteilung aufweisen.
d) Als **amphotere bzw. zwitterionische Tenside** kommen beispielsweise Alkylbetaine, Alkylamidobetaine, Aminopropionate, Aminoglycinate, Imidazoliniumbetaine und/oder Sulfobetaine in Betracht.
   Typische Beispiele sind die Umsetzungsprodukte von primären bzw. tertiären Fettaminen, Fettsäureamiden, Fettsäureaminoaminen oder Fettalkylimidazolinen mit Natriumchloracetat, Acrylsäureester oder Chlorhydroxypropansulfonsäure. Zu Herstellung und Struktur der genannten Verbindungen sei auf **Falbe (ed.), "Surfactants in consumer products", Springer-Verlag, 1986, S.114-119** verwiesen. Vorzugsweise werden Alkylamidobetaine eingesetzt, die durch Kondensation von technischer C_{12/14}-bzw. C_{12/18}-Kokosfettsäure mit Dimethylaminopropylamin und nachfolgende Reaktion mit Natriumchloracetat erhalten werden.

Des weiteren hat es sich als besonders vorteilhaft erwiesen - innerhalb der vorgegebenen Grenzen -
o Fettalkoholsulfate und Fettalkoholethersulfate im Gewichtsverhältnis 34:66,
o die Alkyl- und/oder Alkenyloligoglykoside und die amphoteren bzw. zwitterionischen Tenside im Gewicht»verhältnis 50 : 50 und
o die Fettalkoholsulfate/Fettalkoholethersulfate einerseits sowie die Alkyl- und/oder Alkenyloligoglykoside/ amphoteren bzw. zwitterionischen Tenside andererseits im Gewichtsverhältnis 70 : 30
einzusetzen.

Bei der Bemessung des Gewichtsverhältnisses von Fettalkoholsulfat und Fettalkoholethersulfat ist dem Umstand Rechnung zu tragen, daß FES im allgemeinen noch Anteile an korrespondierenden FAS enthalten.

Die Herstellung der wäßrigen Detergensgemische kann durch einfaches mechanisches Vermischen der wäßrigen Lösungen der Komponenten, gegebenenfalls bei erhöhten Temperaturen von 30 bis 50°C erfolgen; eine chemische Reaktion findet hierbei nicht statt. Der Feststoffgehalt der wäßrigen Detergensgemische kann 15 bis 50, vorzugsweise 20 bis 40 Gew.-% beträgen.

Die Erfindung wird beispielhaft illustriert an Hand von Handgeschirrspülmitteln, enthaltend - jeweils bezogen auf den Feststoffgehalt -
a) 12 bis 15 Gew.-% Alkyl- und/oder Alkenyloligoglykoside,
b) 20 bis 30 Gew.-% Alkylsulfate,
c) mindestens 40 Gew.-% Alkylethersulfate und
d) 12 bis 15 Gew.-% amphotere bzw. zwitterionische Tenside.

Neben den genannten Detergensgemischen können die wäßrigen Handgeschirrmittel weitere übliche Bestandteile, beispielsweise weitere anionische, nichtionische oder amphotere bzw. zwitterionische Cotenside, Schaumbooster, Duftstoffe etc. aufweisen. Eine typische Rezeptur kann beispielsweise 47 Gew.-% FES, 23 Gew.-% FAS und jeweils 15 Gew.-% APG und Alkylamidobetain - jeweils bezogen auf den Feststoffgehalt des Mittels - enthalten. Der Feststoffgehalt der Handgeschirrspülmittel kann 15 bis 50, vorzugsweise 20 bis 40 Gew.-% betragen.

### Gewerbliche Anwendbarkeit

Die erfindungsgemäßen Detergensgemische zeichnen sich durch ausgezeichnete Wasch-, Spül- und Reinigungsleistung sowie eine vorteilhafte hautkosmetische und ökotoxikologische Verträglichkeit aus.

Sie eignen sich zur Herstellung von Wasch-, Spül- und Reinigungsmitteln, Produkten zur Haar- und Körperpflege, in denen sie in Mengen von 1 bis 50, vorzugsweise 10 bis 30 Gew.-% - bezogen auf die Mittel - enthalten sein können.

Die folgenden Beispiele sollen den Gegenstand der Erfindung näher erläutern, ohne ihn darauf einzuschränken.

### Beispiele

### I. Eingesetzte Tenside

- APG:: C_{12/14}-Kokosalkyloligoglucosid
Plantaren^{(R)} APG 600 (30 gew.-%ige wäßrige Paste)
- FAS:: C_{12/14}-Kokosfettalkoholsulfat
Texapon^{(R)} LS35 (35 gew.-%ige wäßrige Paste)
- FES:: C_{12/14}-Kokosfettalkohol-2EO-Addukt-Sulfat-Na-Salz
Texapon^{(R)} NSO (28 gew.-%ige wäßrige Lösung)
- AMP:: Betaintensid auf Basis C_{12/14}-Kokosfettsäureamid
Dehyton^{(R)} K

Alle eingesetzten Tenside sind Verkaufsprodukte der Henkel KGaA, Düsseldorf/FRG.

### II. Beurteilung des Tellerspülvermögens (TSV)

Die Ermittlung des Tellerspülvermögens wurde mit Hilfe des Teller-Testes [**Fette, Seifen, Anstrichmitt., 74, 163 (1972)**] durchgeführt. Hierzu wurden Teller mit einem Durchmesser von 14 cm mit je 2 cm³ Rindertalg (Säurezahl 9-10) angeschmutzt und 24 h bei Raumtemperatur gelagert. Anschließend wurden die Teller bei 50°C mit 5 1 Leitungswasser der Härte 16°d gespült. Die Prüfmischung wurde mit einer Dosierung von 0,15 g Aktivsubstanz/l eingesetzt. Der Spülversuch wurde abgebrochen, sobald der Schaum vollständig verschwunden war. Die Ergebnisse der Spülversuche sind in Tab.1 zusammengefaßt; angegeben ist die Anzahl gespülter Teller.

**Tab.1:**

| Anwendungstechnische Untersuchungen Prozentangaben als Gew.-% | | | | | |
|---|---|---|---|---|---|
| Bsp. | APG % | FAS % | FES % | AMP % | TSV % |
| 1 | 15 | 23 | 47 | 15 | 16 |
| V1 | 0 | 34 | 66 | 0 | 10,5 |
| V2 | 5 | 30 | 60 | 5 | 13,5 |
| V3 | 10 | 27 | 53 | 10 | 15,0 |
| V4 | 20 | 20 | 40 | 20 | 13,5 |

V1, V2, V3 und V4 sind Vergleichsbeispiele; sie fallen nicht in den Bereich der Ansprüche.

## Patentansprüche

1. Wäßrige Detergensgemische, enthaltend - jeweils bezogen auf den Feststoffgehalt -
a) 12 bis 15 Gew.-% Alkyl- und/oder Alkenyloligoglykoside
b) 20 bis 30 Gew.-% Alkylsulfate,
c) mindestens 40 Gew.-% Alkylethersulfate und
d) 12 bis 15 Gew.-% amphotere bzw. zwitterionische Tenside

2. Wäßrige Detergensgemische nach Anspruch 1, **dadurch gekennzeichnet,** daß sie Alkyl- und/oder Alkenyloligoglykoside der Formel (I) enthalten,
R¹-O-[G]ₚ (I)
in der R¹ für einen linearen oder verzweigten Alkyl- und/oder Alkenylrest mit 4 bis 22 Kohlenstoffatomen, [G] für eine Glykoseeinheit mit 5 oder 6 Kohlenstoffatomen und p für eine Zahl von 1 bis 10 steht.

3. Wäßrige Detergensgemische nach den Ansprüchen 1 und 2, **dadurch gekennzeichnet,** daß sie Alkylsulfate der Formel (II) enthalten,
R²O-SO₃X (II)
in der R² für einen linearen oder verzweigten Alkyl- und/oder Alkenylrest mit 6 bis 22 Kohlenstoffatomen und X für ein Alkali- oder Erdalkalimetall steht.

4. Wäßrige Detergensgemische nach den Ansprüchen 1 bis 3, **dadurch gekennzeichnet,** daß sie Fettalkoholethersulfate mit eingeengter Homologenverteilung der Formel (III) enthalten,
R³O-(CH₂CH₂O)ₙ-SO₃X (III)
in der R³ für einen linearen oder verzweigten Alkyl- und/oder Alkenylrest mit 6 bis 22 Kohlenstoffatomen, n für Zahlen von 1 bis 10 und X für ein Alkali- oder Erdalkalimetall steht.

5. Wäßrige Detergensgemische nach den Ansprüchen 1 bis 4, **dadurch gekennzeichnet,** daß sie amphotere bzw. zwitterionische Tenside enthalten, ausgewählt aus der Gruppe, die gebildet wird von Alkylbetainen, Alkylamidobetainen, Aminopropionaten, Aminoglycinaten, Imidazoliniumbetainen und Sulfobetainen.

6. Wäßrige Detergensgemische nach den Ansprüchen 1 bis 5, **dadurch gekennzeichnet,** daß sie einen Feststoffgehalt von 15 bis 50 Gew.-% aufweisen.

## Claims

1. Water-containing detergent mixtures comprising - based on the solids content -
a) 12 to 15% by weight of alkyl and/or alkenyl oligoglycosides,
b) 20 to 30% by weight of alkyl sulfates,
c) at least 40% by weight of alkyl ether sulfates and
d) 12 to 15% by weight of amphoteric or zwitterionic surfactants.

2. Water-containing detergent mixtures as claimed in claim 1, **characterized in that** they contain alkyl and/or alkenyl oligoglycosides corresponding to formula **(I)**:
R¹-O-[G]ₚ (I)
in which R¹ is a linear or branched alkyl and/or alkenyl radical containing 4 to 22 carbon atoms, [G] is a glycose unit containing 5 or 6 carbon atoms and p is a number of 1 to 10.

3. Water-containing detergent mixtures as claimed in claims 1 and 2, **characterized in that** they contain alkyl sulfates corresponding to formula **(II)**:
R²O-SO₃X (II)
in which R² is a linear or branched alkyl and/or alkenyl radical containing 6 to 22 carbon atoms and X is an alkali metal or alkaline earth metal.

4. Water-containing detergent mixtures as claimed in claims 1 to 3, **characterized in that** they contain narrow-range fatty alcohol ether sulfates corresponding to formula **(III)**:
R³O-(CH₂CH₂O)ₙ-SO₃X (III)
in which R³ is a linear or branched alkyl and/or alkenyl radical containing 6 to 22 carbon atoms, n is a number of 1 to 10 and X is an alkali metal or alkaline earth metal.

5. Water-containing detergent mixtures as claimed in claim 1, **characterized in that** they contain amphoteric or zwitterionic surfactants selected from the group consisting of alkyl betaines, alkyl amidobetaines, aminopropionates, aminoglycinates, imidazolinium betaines and sulfobetaines.

6. Water-containing detergent mixtures as claimed in claims 1 to 5, **characterized in that** they have a solids content of 15 to 50% by weight.

## Revendications

1. Mélanges aqueux de détergents, contenant, par rapport respectivement à la teneur en matières solides :
a) 12 à 15 % en poids d'alkyle et/ou alcényloligoglycosides,
b) 20 à 30 % en poids d'alkylsulfates,
c) au moins 40 à 60 % en poids d'alkyléthersulfates et,
d) 12 à 15 % en poids de tensioactifs amphotères ou zwitterioniques.

2. Mélanges aqueux de détergents selon la revendication 1, caractérisés en ce qu'
ils contiennent des alkyles et/ou alcényloligoglycosides de formule (I),
R¹-O-[G]ₚ (I)
où R¹ est un radical linéaire ou ramifié alkyle et/ou alcényle de 4 à 22 atomes de carbone, [G] est une unité glucose de 5 ou 6 atomes de carbone et p est un nombre de 1 à 10.

3. Mélanges aqueux de détergents selon les revendications 1 et 2,
caractérisés en ce qu'
ils contiennent des alkylsulfates de formule (II),
R²O-SO₃X (II)
où R² est un radical linéaire ou ramifié alkyle et/ou alcényle avec 6 à 22 atomes de carbone et X est un métal alcalin ou alcalino-terreux.

4. Mélanges aqueux de détergents selon les revendications 1 à 3,
caractérisés en ce que
ils contiennent des éthersulfates d'alcools gras à répartition étroite en homologues de formule (III),
R³O-(CH₂CH₂O)ₙ-SO₃X (III)
où R³ représente un radical linéaire ou ramifié alkyle et/ou alcényle de 6 à 22 atomes de carbone, n représente des nombres de 1 à 10 et X est un métal alcalin ou alcalino-terreux.

5. Mélanges aqueux de détergents selon les revendications 1 à 4,
caractérisés en ce que
ils contiennent des tensioactifs amphotères ou zwitterioniques, choisis dans le groupe formé par des alkylbétaïnes, alkylamidobétaïnes, aminopropionates, aminoglycinates, imidazoliniumbétaïnes et sulfobétaïnes.

6. Mélanges aqueux de détergents selon les revendications 1 à 5,
caractérisés en ce que
ils présentent une teneur en matières solides de 15 à 50 % en poids.
